# EUROPÄISCHE PATENTSCHRIFT

(11) **EP 3 946 066 B1**
(45) Veröffentlichungstag und Bekanntmachung des Hinweises auf die Patenterteilung: **07.10.2026**
(21) Anmeldenummer: 20716735.4
(22) Anmeldetag: 30.03.2020
(51) Int. Cl.: A61B 8/08, A61B 8/00, A61B 8/15

(54) **VERFAHREN ZUR HERSTELLUNG EINES GEL-FORMKÖRPERS**
METHOD FOR PRODUCING A SHAPED GEL BODY
PROCÉDÉ POUR FABRIQUER UN CORPS MOULÉ SOUS FORME DE GEL

(30) Priorität: 29.03.2019 DE 102019204522
(43) Veröffentlichungstag der Anmeldung: 09.02.2022
(73) Patentinhaber: Lechner, Johann, 81547 München (DE)
(72) Erfinder: ZIMMERMANN, Bernd, 82319 Starnberg (DE); STURM, Michael, 82234 Weßling (DE); LECHNER, Johann, 81547 München (DE)
(74) Vertreter: Bardehle Pagenberg Partnerschaft mbB Patentanwälte Rechtsanwälte
(86) Internationale Anmeldenummer: PCT/EP2020/058962
(87) Internationale Veröffentlichungsnummer: WO 2020/201216

(56) Entgegenhaltungen:
- WO-A1-90/01902
- US-A- 6 039 694
- US-A1- 2016 199 027

## Beschreibung

### Gebiet der Erfindung

Die Erfindung betrifft die Herstellung eines Gel-Formkörpers. Insbesondere wird der Gel-Formkörper eingesetzt, um dentale Kavitäten im Kieferknochen durch Transmissions-Alveolar-Ultraschallmessungen (TAU) nachzuweisen und zu lokalisieren.

### Hintergrund der Erfindung

Im Bereich der Medizin wird die Puls-Echo-Sonographie in großem Umfang eingesetzt, um alle Arten von weichem Gewebe bildlich darzustellen. Grundsätzlich werden Strukturen im Inneren des Körpers abgebildet, indem Reflexionen von Ultraschallwellen ausgewertet werden. Diese Methode ist jedoch nicht brauchbar, um nützliche Informationen vom Zustand des Kieferknochens zu erhalten, wegen der fast vollständigen Reflexion von Ultraschall an der Grenzfläche zwischen Knochen und weichem Gewebe. Insbesondere kann der spongiöse Teil des Kieferknochens mit üblicherweise verwendeter Ultraschallausrüstung nicht untersucht werden. Deshalb ist bis heute Ultraschall von sehr begrenztem Nutzen in der Zahnmedizin.

Der Zustand der Spongiosa im Kiefer kann klinisch von großer Bedeutung sein. *Jerry Bouquot* hat anatomische Beweise vorgelegt, dass der spongiöse Teil des Kiefers in großem Umfang degeneriert sein kann, ein Phänomen, das er unter anderem "ischemic *osteonecrosis leading to cavitational lesions"* also "ischämische Osteonekrose, die zu Schäden durch Hohlraumbildung führt" nennt. Er bringt die Osteonekrose des Kieferknochens mit neuralgischen Schmerzen in Verbindung und definiert eine Erkrankung genannt *"neuralgia inducing cavitational osteonecrosis (NICO)",* also "Neuralgie induzierende hohlraumbildende Osteonekrosen (NICO)" - (vgl. J. E. Bouquot, A. M. Roberts, P. Person and J.Christian, "Neuralgia-inducing cavitational osteonecrosis (NICO). Osteomyelitis in 224 jawbone samples from patients with facial neuralgia", Oral Surg Oral Med Oral Pathol. 1992, 73 (3):307-319; J. Bouquot, W. Martin and G. Wrobleski "Computer-based thru-transmission sonography (CTS) imaging of ischemic osteonecrosis of the jaws - a preliminary investigation of 6 cadaver jaws and 15 pain patients", Oral Surg Oral Med Oral Pathol Oral Radiol Endod. 2001, 92: 550.)

*Johann Lechner* untersuchte intensiv das Gewebe in solchen geschädigten Bereichen, das sich als Fettklumpen im Inneren einer intakten Kortikalis darstellt. Bei einer Operation wird dieses Material einfach ausgelöffelt. Dieses Gewebe ist in einem ischämischen, fettig-degenerierten Zustand. *Lechner* definiert deshalb die beobachteten Veränderungen als "fettig-degenerative Osteolyse/Osteonekrose des Kieferknochens (FDOK)", im Englischen als "fatty-degenerative osteolysis/osteonecrosis of jawbone (FDOJ)" bezeichnet. Er zeigte, dass diese Fettklumpen, die im Kieferknochen gefunden werden, biochemisch außerordentlich aktiv sind, indem sie bestimmte Zytokine in großen Mengen produzieren, namentlich RANTES (CCL5) und FGF-2, aber auch PDGF und MCP-1. Die Konzentration dieser Zytokine ist auch bei einer Anzahl von systemischen Krankheiten erhöht, wie bei Krebs, Demenz, multipler Sklerose oder Arthritis. Es gibt starke Hinweise - aufgrund von aufsehenerregenden klinischen Verbesserungen nach operativer Entfernung solcher Gewebe - darauf, dass die Entwicklung und das Fortdauern einer Vielfalt von systemischen Erkrankungen mit der fettig-degenerativen Osteolyse/Osteonekrose des Kieferknochens in Beziehung steht. Jedoch fehlt in den meisten Fällen der lokale Effekt des neuralgischen Schmerzes (NICO). (Vgl. J. Lechner and V. von Baehr, "RANTES and fibroblast growth factor 2 in jawbone cavitations: triggers for systemic disease?" International Journal of General Medicine 2013, 6: 277-290; J. Lechner and W. Mayer "Immune messengers in neuralgia inducing cavitational osteonecrosis (NICO) in jaw bone and systemic interference", Eur. J. Integr. Med. 2010, 2 (2): 71-77.)

Zudem ist es nach einer kürzlich erschienen Veröffentlichung plausibel, dass NICO und FDOK ebenso wie die sogenannte "aseptic ischemic osteonecrosis in the jawbone" (AIOJ), also die aseptische, ischämische Osteonekrose im Kieferknochen, alle denselben pathologischen Zustand des Kieferknochens beschreiben, der unter dem Code M87.0 der Internationalen statistischen Klassifikation von Krankheiten und verwandten Gesundheitsproblemen, 10. Version (ICD-10) aufgeführt ist. (Vgl. J. Lechner, S. Schuett and V. von Baehr, "Aseptic-avascular osteonecrosis: local 'silent inflammation' in the jawbone and RANTES/CCL5 overexpression", Clinical, Cosmetic and Investigational Dentistry 2017:9 99-109.)

Außerdem ist der Zustand des spongiösen Anteils des Kieferknochens von großer Bedeutung für zahnärztliche Implantate und für den Erfolg der Implantologie, entsprechend einer früheren Veröffentlichung *Bilal AI-Nawas.* (Vgl. M.O. Klein, K.A. Grotz, B. Manefeld, P.H. Kann and B. Al-Nawas, "Ultrasound transmission velocity for non-invasive evaluation of jaw bone quality in vivo prior to dental implantation", Ultrasound in Medicine & Biology 2008, 34: 1966-1971.)

Es können also schwerwiegende gesundheitliche Risiken mit einer fettig-degenerativen Osteolyse des Kieferknochens verbunden sein. Ein bedeutendes Problem dabei ist aber, dass ein Kieferknochen mit fettig-degenerativer Osteolyse im Röntgenbild keine ungewöhnlichen Befunde liefert. Das gilt selbst dann, wenn der Kieferknochen in einem weitgehend degenerierten Zustand ist und nur noch Fettgewebe anstelle der *substantia spongiosa* eines gesunden spongiösen Knochens (FDOK) aufweist. Das Auftreten und die Phänomene von AIOJ, FDOK und NICO bleiben weitgehend unbekannt und werden sogar bestritten oder verneint, weil sie bei jedweder Röntgenuntersuchung nicht nachweisbar sind. (Vgl. J. Lechner, "Validation of dental X-ray by cytokine RANTES - comparison of X-ray findings with cytokine overexpression in jawbone", Clinical, Cosmetic and Investigational Dentistry 2014, 6: 71-79.)

Um das oben beschriebene Problem zu überwinden, war eine andere Herangehensweise notwendig. Anstelle von Röntgenaufnahmen oder anderer etablierter medizinischer Untersuchungsmethoden wurde die Verwendung von Transmissions-Alveolar-Ultraschallmessungen (TAU) ausgewertet. Transmissions-Ultraschallmessungen sind in einigen technischen Bereichen wie der Luftfahrtindustrie wohlbekannt, z.B. zur Untersuchung von Schweißnähten. Diese Untersuchungsmethode wird aber auch im medizinischen Bereich verwendet zur Bestimmung der Knochendichte bei der Diagnose von Osteoporose. (Vgl. C. M. Langton and C. F. Njeh. "The Measurement of Broadband Ultrasonic Attenuation in Cancellous Bone-A Review of the Science and Technology", IEEE Transactions on Ultrasonics, Ferroelectrics, and Frequency Control, 2008 (55) 7: 1546-1554.)

US 6,030,221 A dürfte die erste Offenbarung einer Vorrichtung zur Transmissions-Alveolar-Ultraschallmessung (TAU) sein, um Kavitäten im Kieferknochen nachzuweisen. Diese TAU-Vorrichtung erzeugt einen Ultraschallpuls und schickt diesen Puls durch den Kieferkochen eines Patienten. Der Ultraschallpuls wird dann von einer Ultraschallempfängereinheit gemessen und aufgezeichnet. Abschwächungen der Amplitude des Ultraschallpulses (Dämpfung) zeigen pathologische Veränderungen im Kieferknochen an. Die Ergebnisse werden auf einem Farbmonitor dargestellt und zeigen unterschiedliche Farben entsprechend unterschiedlichen Graden der Dämpfung.

Die TAU-Vorrichtung entsprechend der US 6,030,221 A war für einige Jahre kommerziell erhältlich. Es handelte sich um den Cavitat Ultrasonograph CAV 4000, entwickelt von Ingenieuren bei Cavitat Medical Technologies. Diese bekannte TAU-Vorrichtung verwendete eine Vielzahl von Empfängerelementen, wobei die Frequenz des ausgesendeten Ultraschallpulses und der Schallwelle im Bereich von etwa 2,5 MHz war. Die Verarbeitung der empfangenen Schallwellen wurde durch einen Digitalumsetzer mit einer Arbeitsfrequenz von etwa 100 MHz vorgenommen. *Jacques Imbeau* beschreibt ausführlicher die zugrundeliegenden Prinzipien und die Handhabung dieser Vorrichtung in der Veröffentlichung J. Imbeau, "Introduction to Through-Transmission Alveolar Ultrasonography (TAU) in Dental Medicine", The Journal of Craniomandibular Practice, 2005 (23) 2: 100-112.

Die vorstehend genannte TAU-Vorrichtung weist eine Reihe von Nachteilen auf, die von *Imbeau,* a.a.O., S. 106/107 näher erläutert werden. In der deutschen Patentanmeldung DE 10 2017 011 311.4 wird nun eine neue TAU-Vorrichtung beschrieben, die diese Nachteile überwindet. Das betrifft die Schwierigkeit, Ultraschallsender und -empfänger reproduzierbar und einfach zu positionieren; es betrifft weiterhin die geringe Empfindlichkeit der alten Cavitat-Vorrichtung, sodann die mechanische Instabilität des Ultraschall-Empfängers und schließlich die fehlende Möglichkeit, das Gerät zu kalibrieren.

Kritisch ist bei allen Ultraschall-Messungen die sichere akustische Kopplung von Messvorrichtung und zu messendem Objekt. Besonders schwierig ist diese Koppelung bei unregelmäßig geformten Objekten, wie sie beispielsweise im Kieferbereich vorliegen. Aufgabe der vorliegenden Erfindung ist es daher, unterschiedliche Abstände zwischen dem Alveolarkamm und den Flächen von Ultraschallsender und/oder-empfänger auszugleichen. Zudem soll die Erfindung eine rasche Anpassung der Position von Ultraschallsender und/oder Ultraschallempfänger erlauben. Damit soll die Handhabung des Gel-Formkörpers vereinfacht und besonders sicher gemacht werden, unter nochmaliger Verbesserung der Reproduzierbarkeit der Messungen.

US 6 039 694 A offenbart eine homogene, feste, elastische, biokompatible Hülle, die sich an den diagnostischen und/oder therapeutischen medizinischen Ultraschallwandler anschmiegt und so wirkt, als wäre sie ein integraler Bestandteil davon, und die aus 20 bis 95 % biokompatibler Flüssigkeit, vorzugsweise etwa 70 bis 95 % biokompatibler Flüssigkeit, besteht, die der Hülle Eigenschaften verleiht, die zu dem gewünschten Maß an akustischer Kopplung führen, mit akzeptablen niedrigen Werten von akustischen Artefakten, Verzerrung und Dämpfung, und die eine mikrobielle Barriere zwischen dem Wandler und einem chirurgischen Feld oder der Haut bereitstellt.

US 2016/199027 A1 offenbart Koppler für Ultraschallsonden, die einen massiven Kopplerkörper mit einem Hohlraum haben können, der eine verformbare Form hat, so dass er sich an das Ende der Sonde anpassen und/oder selbst daran befestigen kann, indem ein Benutzer den Kopplerkörper gegen die Ultraschallsonde drückt, so dass der Kopplerkörper die darunter liegende Form der Ultraschallsonde annimmt, während der äußere Körper seine vormontierte Form beibehalten kann.

WO 90/01902 A1 offenbart einen mit einer Ultraschallsonde kombinierten Akustikkoppler, der aus einem Gel besteht, von dem ein Teil gehärtet ist, um ein Haltemittel zum Halten der Sonde bereitzustellen, um die Sonde mit dem Koppler anzuheben, und von dem ein anderer Teil verwendet wird, um einen akustisch transparenten Pfad zwischen der Sonde und einer Oberfläche eines akustisch zu untersuchenden Objekts herzustellen.

Zur Lösung dieser Aufgabe beschreibt die vorliegende Erfindung die Herstellung eines Gel-Formkörpers nach Anspruch 1.

Der Gel-Formkörper ist bevorzugt quaderförmig oder ist von einem Quader abgeleitet und weist einen Innenraum, eine Aussparung, einen Einschnitt, Schlitz oder Ähnliches (im Folgenden "Aufnahme") auf zur Aufnahme eines Ultraschallsenders und/oder - empfängers. Die Kanten des Quaders sind bevorzugt gefast oder gerundet. Vorzugsweise kann mindestens eine der schmalen Seitenflächen gerundet sein und/oder es sind die Kanten von Boden- und Deckfläche paarweise nach außen gewölbt. Der Gel-Formkörper und die vorgesehene Aufnahme werden bevorzugt an die Form von Ultraschallsender und/oder -empfänger angepasst. Es können aber auch beliebig geformte Ausgangsformkörper mit einer Aufnahme zur Aufnahme eines Ultraschallsenders und/oder -empfängers versehen sein.

Zur leichteren Handhabung wird die Aufnahme mit ausreichenden Mengen eines nicht-formstabilen Ultraschallgels befüllt. Weiterhin können der oder die Gel-Formkörper in eine flexible, biokompatible Schutzhülle eingeführt sein, die ebenfalls mit einem nicht-formstabilen Ultraschallgel gefüllt sein kann.

Zur Herstellung des Gel-Formkörper werden Quader eines formstabilen Gels in passender Größe über die schmalen Seitenflächen des Quaders aufgeschnitten, vorzugsweise aber nicht ganz durchgeschnitten und zur Herstellung der Aufnahme nur über drei der vier Seiten oder bei nicht vollständigem Aufschneiden nur über zwei der vier Seiten an den Rändern geschlossen bzw. verklebt.

Die Gel-Formkörper, gegebenenfalls in Schutzhüllen, werden für den ärztlichen oder zahnärztlichen Gebrauch eingesetzt, bevorzugt als Einwegartikel und insbesondere bei Transmissions-Alveolar-Ultraschallmessungen zur Lokalisierung von Kavitäten im Kieferknochen. Besonders bevorzugt ist die Verwendung mit einer Vorrichtung und für ein Verfahren nach DE 10 2017 011 311.4. Somit wird durch die Erfindung die Lehre der DE 10 2017 011 311.4 hinsichtlich der Verwendung von Gel-Formkörpern fortentwickelt.

### Kurze Erläuterung der Zeichnungen

Figur 1 zeigt einen Gel-Formkörper (1) mit Aufnahme (2);
Figur 2 zeigt einen Gel-Formkörper mit gerundeter Seitenfläche (4);
Figur 3 zeigt einen Gel-Formkörper, bei dem die Kanten von Boden- und Deckfläche (5) paarweise nach außen gewölbt sind;
Figur 4 zeigt ein gebrauchsfertiges System aus Gel-Formkörper (1), Schutzhülle (6) und Ultraschallsender oder-empfänger (7);
Figur 5 zeigt zwei Beispiele des Gel-Formkörpers (1), bei denen sowohl die schmalen Seitenflächen gerundet als auch Kanten von Boden- und Deckfläche paarweise nach außen gewölbt sind;
Figur 6zeigt eine maßstabsgetreue Plastiktüte zum Transport Gel-Formkörpers (1) in einer Schutzhülle (6) gemäß Figur 7; und
Figur 7 zeigt einen maßstabsgetreuen, gebrauchsfertigen Gel-Formkörper (1) in einer Schutzhülle (6).

### Detaillierte Beschreibung von bevorzugten Ausführungsformen der Erfindung

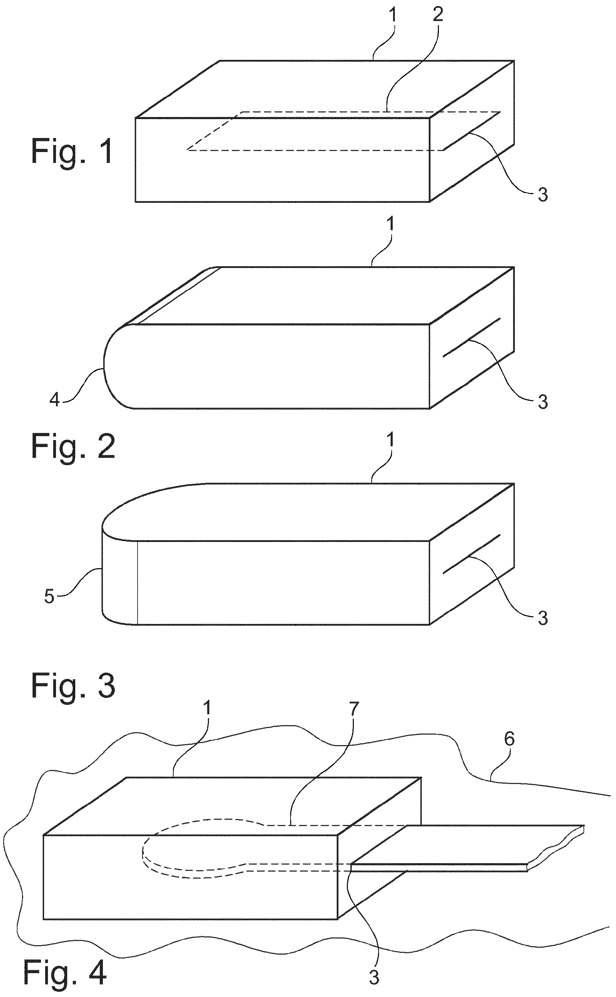

**Figur** 1 zeigt einen Gel-Formkörper (1) zur Verwendung bei Ultraschallmessungen, bei dem die vorgesehene Aufnahme (2) gestrichelt dargestellt ist. Die Aufnahme (2) kann aufgrund der Elastizität des Gel-Formkörpers als einfacher Einschnitt bzw. als Schlitz ausgeführt werden, der auf der Seitenfläche mit dem Bezugszeichen (3) gekennzeichnet ist; ein Ultraschallsender und/oder -empfänger kann/können einfach durch den Einschnitt (3) in die Aufnahme (2) geschoben werden. Der elastische Gel-Formkörper (1) ist vorzugsweise im Wesentlichen quaderförmig und weist eine Boden- und eine Deckfläche sowie vier schmalere Seitenflächen auf.

Der Gel-Formkörperkann kann von einem Quader auch derart abgeleitet sein, dass mindestens eine der schmalen Seitenflächen gerundet (4) ist, wie in **Figur** 2 gezeigt.

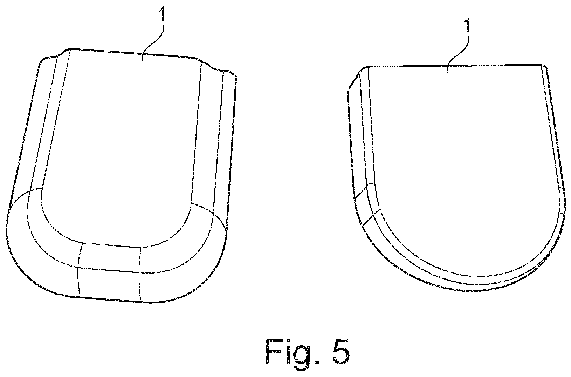

Eine alternative Rundung (5) zeigt **Figur** 3. In besonders bevorzugten Ausführungsformen sind beide Rundungsformen vereint, wie in **Figur** 5 gezeigt.

Im Inneren des Gel-Formkörpers ist eine Aufnahme (2) vorgesehen, der einen für die Ultraschallmessungen vorgesehenen Sender und/oder Empfänger (7) aufnehmen kann, wobei die Aufnahme (2) eine Öffnung (3) nach außen aufweist, um Sender oder Empfänger (7) einführen zu können und um Verbindungselemente des Ultraschallsenders und/oder -empfängers (7) aufzunehmen.

Der Gel-Formkörper (1) weist insbesondere bei kugelförmiger Gestalt einen Durchmesser zwischen 1 cm und 10 cm auf, bevorzugt zwischen 1,5 cm und 7 cm, besonders bevorzugt zwischen 2 cm und 5 cm liegt und/oder bei im Wesentlichen quaderförmiger Gestalt eine Länge der Boden- oder Deckfläche von 1 cm bis 10 cm, bevorzugt 2 cm bis 8 cm und besonders bevorzugt 2,5 cm bis 5,0 cm beträgt, eine Breite der Boden- oder Deckfläche von 1 cm bis 8 cm, bevorzugt 1,5 cm bis 6,0 cm, besonders bevorzugt 2 cm bis 4 cm beträgt und ganz besonders bevorzugt 3,0 cm beträgt und/oder eine Höhe von 0,8 cm bis 4 cm, bevorzugt 1 cm bis 3 cm und besonders bevorzugt 1,0 cm bis 1,4 cm.

Ein derartiger Gel-Formkörper (1) ist einfach herzustellen, sicher und einfach in der Handhabung und liefert eine gute akustische Ankopplung und damit die Voraussetzung für gute Messergebnisse.

Die Messergebnisse werden verbessert, wenn der Gel-Formkörper (1) keine scharfen Kanten und Ecken aufweist. Um das erreichen, werden die Ecken und Außenkanten des Gel-Formkörpers (1) bevorzugt gefast oder gerundet.

Es ist zudem bevorzugt, wenn die äußere Gestalt des Gel-Formkörpers (1) der Form des aufzunehmenden Ultraschallsender und/oder -empfängers (7) angepasst ist. Ebenfalls bevorzugt ist es, wenn die vorgesehene Aufnahme (2) der Form des aufzunehmenden Ultraschallsender und/oder -empfängers (7) angepasst ist.

Es hat sich aus ultraschalltechnischen Gründen als günstig erwiesen, den Ultraschallsender größer als den Ultraschallempfänger zu dimensionieren, so dass auch die zugehörigen Gel-Formkörper unterschiedlich groß gefertigt werden können. Bevorzugt ist es auch, bei dem für einen Ultraschallsender vorgesehenen Gel-Formkörper zumindest eine Gelschicht dicker auszuführen als die Gelschichten bei dem Gel-Formkörper, der für den Ultraschallempfänger vorgesehen ist. Eine mögliche Ausführungsform sieht 7 mm und 4 mm für die Dicke der Gelschichten vor, die im Weg des Ultraschalls liegen.

In der Praxis der TAU-Messungen nach DE 10 2017 011 311.4 hat es sich als vorteilhaft erwiesen, den Ultraschallempfänger enoral, also im Inneren des Mundes, einzusetzen und den Ultraschallsender außen auf der Wange des Patienten zu positionieren und gegebenenfalls mit Hilfe von etwas Ultraschallgel eine gute akustische Ankoppelung herzustellen. Bei schwierigen anatomischen Gegebenheiten im Inneren des Mundes kann es jedoch geschehen, dass der Ultraschallempfänger nur in einer einzigen Position gute Messergebnisse liefert. Das kann aber unter Umständen bedeuten, dass dann der Ultraschallsender keine ausreichende akustische Ankoppelung mehr ermöglicht, es sei denn, dass für den Ultraschallsender - wie hier gelehrt - eine dickere Gelschicht vorgesehen ist, die entsprechend stärker verformt werden kann, als es bei dünnen Gelschichten möglich ist, ohne die akustische Ankoppelung zu verlieren.

Bei Messungen im Kieferbereich ist es vorteilhaft, dass die Ultraschallsender und - empfänger (7) ihrerseits keine scharfen Ecken und Kanten aufweisen, um bei der Anwendung beim Patienten Missempfindungen oder Schmerzen zu vermeiden. Deshalb wird die äußere Gestalt der Ultraschall-Messelemente bevorzugt gerundet, wie in **Figur 4** gezeigt. Daraus ergibt sich, dass die vorgesehene Aufnahme (2) - bevorzugt in dem der Öffnung (3) gegenüberliegenden Bereich - auch gerundet ist. Weiterhin ist es bevorzugt, dass eine gerundete (4) Seitenfläche der Öffnung (3) gegenüberliegt und/oder dass Kanten von Boden- und Deckfläche paarweise nach außen gewölbt (5) sind und der Öffnung (3) gegenüberliegen.

Bei der Puls-Echo-Sonographie im Stand der Technik ist z.B. über Knochen oder Gelenken die Verwendung einer Gelschicht, einer sogenannten Vorlaufstrecke, aus schalltechnischen Gründen erforderlich. Eine solche Gelschicht kann auf einfache Weise dadurch bereitgestellt werden, dass für einen beliebig geformten Ultraschallkopf ein Gel-Formkörper (1) eingesetzt wird, der im Inneren eine Aufnahme (2) für den Ultraschallkopf aufweist, die von einer Gelschicht umgeben ist, deren Dicke 1,0 cm bis 5,0 cm, bevorzugt 1,2 cm bis 3 cm beträgt.

Bei der Auswahl geeigneter formstabiler, elastischer Gele kann von thermoplastischen Elastomeren ausgegangen werden, bei denen ein polymerer Grundkörper eine geeignete niedermolekulare Flüssigkeit aufnehmen kann. Dabei ist darauf zu achten, dass die akustischen Eigenschaften der Gel-Formkörper die Messungen nicht ungünstig beeinflussen. Das bedeutet, dass der Gel-Formkörper bevorzugt ein formstabiles elastisches Gel umfasst, welches eine Schallgeschwindigkeit im Bereich von 1460 m/s bis 1615 m/s und eine Schalldämpfung im Bereich von 0,3 dB/cm bis 1,5 dB/cm (1 MHz) aufweist.

Geeignete formstabile elastische Gele sollten weich, (sehr) elastisch und (sehr) flexibel sein, so dass immer ein möglichst vollständiger Kontakt des formstabilen elastischen Gels mit dem Ultraschallempfänger und dem Alveolarkamm sichergestellt werden kann. Es hat sich gezeigt, dass all diese Eigenschaften zumindest dann erfüllt sind, wenn das verwendete Gel eine Rückstellgeschwindigkeit für die spontane Rückverformung von höchstens 80 mm/sec aufweist.

Wegen der Flexibilität des Gels ist es auch möglich, die Position der Messeinheit, bestehend aus Ultraschallsender und -empfänger, zu justieren, ohne den Kontakt des Gels und ohne die Messungen zu stören. Die formstabile Eigenschaft des Gels verhindert, dass das Gel vor oder während der Messung zu fließen beginnt und in Bereiche verschwindet, in denen nicht gemessen wird. Das geschieht nämlich leicht bei der Verwendung von nicht formstabilen, üblicherweise verwendeten Ultraschallgelen und bedeutet dort, dass verlässliche Messungen oft nicht durchgeführt werden können. Besonders bei Ultraschallmessungen im Oberkiefer ist die Formstabilität des Gel-Formkörpers (1) von großer Bedeutung, denn im Oberkiefer-Bereich ist die Tendenz zum Wegfließen von Ultraschallgelen aufgrund der Schwerkraft sehr ausgeprägt. Mit den erfindungsgemäßen Gel-Formkörpern (1) lassen sich aber auch im Oberkiefer-Bereich einfach und reproduzierbar TAU-Messungen durchführen, ohne dass weitere Hilfskonstruktionen nötig sind, um das Gel am Platz zu halten.

Formstabile elastische Gele mit den genannten Eigenschaften sind kommerziell erhältlich, z.B. SONOGEL Sonokit soft, Artikelnummern 6510 und 6520, die von der Sonogel Vertriebs-GmbH in Bad Camberg, Deutschland vertrieben werden. Es handelt sich dabei um ein Styrol-Block-Copolymer mit einem hydrogenierten Mittelblock aus Styrol-Ethylen/Butylen-Styrol oder Styrol-Ethylen/Propylen-Styrol und gehört zu der Verbindungsklasse der thermoplastischen Elastomere, mit Paraffinöl als niedermolekularem Bestandteil. Andere geeignete Materialien sind beispielsweise Silikone, gemischt mit Silikonölen oder sehr weichgemachtes Polyvinylchlorid. Um das Einführen von Ultraschallsender und/oder -empfänger (7) in den Gel-Formkörper (1) zu erleichtern, hat es sich bewährt, in die vorgesehene Aufnahme (2) ein üblicherweise bei Ultraschallmessungen verwendetes, nicht-formstabiles Gel (im Folgenden als "Ultraschallgel" bezeichnet) zu füllen. Die Menge an dazu erforderlichem Ultraschallgel ergibt sich aus der Viskosität des Ultraschallgels und den Rückstellkräften des Gel-Formkörpers (1).

Weiterhin ist es aus hygienischen und aus anwendungstechnischen Gründen vorteilhaft, den Gel-Formkörper (1) mit einer flexiblen, möglichst biokompatiblen Schutzhülle (6) zu versehen. **Figur 4** zeigt ein gebrauchsfertiges System aus Gel-Formkörper (1), Schutzhülle (6) und Ultraschallsender oder-empfänger (7) und der Öffnung der Aufnahme (2) nach außen (3). Das nicht-formstabile Ultraschallgel ist nicht dargestellt. Das System umfasst auch die - ebenfalls nicht dargestellte - Anordnung, bei der sowohl der Ultraschallsender als auch der Ultraschallempfänger in die Gel-Formkörper (mit oder ohne Schutzhülle) eingeführt sind.

Mit der Schutzhülle werden der Transport und die Handhabung der Gel-Formkörper sehr erleichtert. Es ist weiterhin möglich, ein Ultraschallgel auch in diese Schutzhülle (6) zu füllen, wobei der Gel-Formkörper (1) ganz oder teilweise von dem Ultraschallgel umgeben ist, je nach der Menge des verwendeten Ultraschallgels. Die Schutzhülle (6) kann beispielsweise aus Polyurethan, Polyethylen oder auch aus Latex sein, jedoch besteht sie bevorzugt aus Polypropylen.

Somit ergibt sich eine sehr einfache Handhabung der Gel-Formkörper (1), denn in einer Zahnarztpraxis müssen beispielsweise lediglich der Ultraschallsender und/oder - empfänger (7) in den Gel-Formkörper (1), der sich in der Schutzhülle (6) befindet, eingeschoben werden, was aufgrund des bereits eingefüllten Ultraschallgels sauber, bequem und rasch machbar ist. Durch Verwendung von Handschuhen sind auch die hygienischen Erfordernisse leicht zu erfüllen, und die Messungen im Mundraum können ohne weitere Vorkehrungen durchgeführt werden.

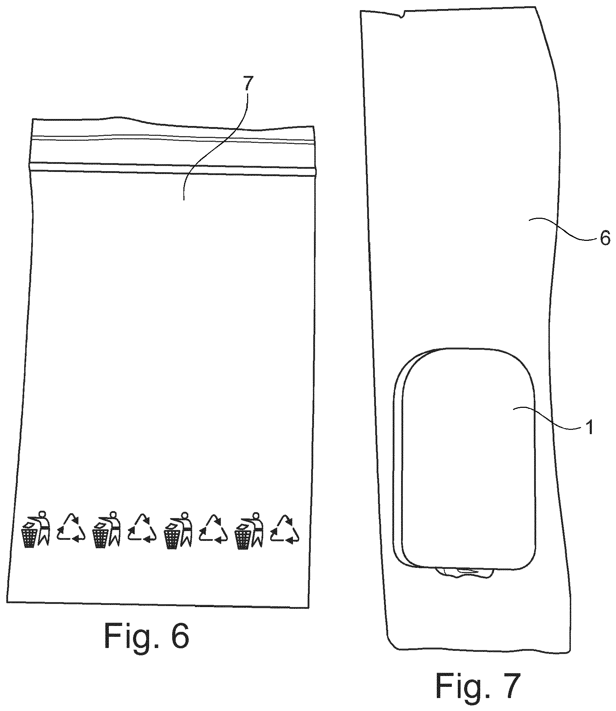

**Figur 7** zeigt maßstabsgetreu den gebrauchsfertigen Gel-Formkörper (1) in einer Schutzhülle (6) neben einer maßstabsgetreuen Plastiktüte zum Transport in **Figur 6****.** Die kritische Abwesenheit von Luftblasen im Gel-Formkörper (1) wird bereits bei der Herstellung geprüft und ist somit Teil der Qualitätskontrolle bei der Herstellung der Gel-Formkörper. Die verwendeten Gel-Formkörper (1) sind also frei von Luftblasen. Wenn beim Einfüllen des Ultraschallgels Luftblasen auftreten, so lassen sich diese Luftblasen leicht erkennen und sie lassen sich durch sanftes Ausstreichen mit dem Finger einfach aus dem Messbereich entfernen. In den Mundraum wird bei diesem Vorgehen kein Ultraschallgel eingeführt, weil es aus toxikologischen und rechtlichen Gründen wenig tolerabel, vor allem aber für den Patienten unangenehm und kaum zumutbar wäre.

Gezeigt ist weiterhin ein System, das aus einem Ultraschallsender und/oder -empfänger (7) besteht, eingeführt in den jeweiligen elastischen Gel-Formkörper (1). Der oder die Gel-Formkörper können sich ihrerseits in einer flexiblen, möglichst biokompatiblen Schutzhülle (6) befinden, die mit einem Ultraschallgel befüllt sein kann. Ein System mit Ultraschallsender und -empfänger (7), Gel-Formkörpern (1) und Schutzhüllen (6), befüllt mit Ultraschallgel, ist unmittelbar für die beabsichtigten Messungen geeignet und vorbereitet.

Gegenstand der Erfindung ist zudem ein Verfahren zur Herstellung eines Gel-Formkörpers (1), bei dem ein Formkörper aufgeschnitten wird und nur teilweise wieder verklebt wird, um die Aufnahme (2) des Gel-Formkörpers (1) zu bilden.

Bei diesem Verfahren wird von einem Formkörper aus einem formstabilen elastischen Gel ausgegangen. Es wird bevorzugt zunächst ein Quader oder ein von einem Quader abgeleiteter Formkörper - mit Boden-, Deck- und Seitenflächen und zugehörigen Außenkanten - zwischen der Boden- und der Deckfläche vollständig oder teilweise aufgeschnitten, um die Bildung der Aufnahme (2) im Gel-Formkörper (1) vorzubereiten. Das Schneiden kann mit einem scharfen Messer oder einer Schere durchgeführt werden.

Bei dem vollständig aufgeschnittenen Formkörper wird die Aufnahme (2) im Inneren des Gel-Formkörpers (1) dadurch gebildet, dass die beiden durch Aufschneiden gebildeten Hälften so aufeinandergelegt werden, dass die ursprüngliche Form des Formkörpers wieder hergestellt wird, und dass der Formkörper an drei Seiten jeweils von den Rändern her durch Verkleben soweit wieder geschlossen wird, dass die verbleibende Aufnahme (2) im Inneren die gewünschte Größe erhält und die erforderliche Öffnung (3) der Aufnahme (2) nach Außen gewährleistet wird.

Der nur teilweise aufgeschnittene Formkörper wird an zwei Seiten jeweils von den Rändern her durch Verkleben soweit wieder geschlossen, dass die Aufnahme (2) im Inneren des Gel-Formkörpers (1) die gewünschte Größe erhält, und die erforderliche Öffnung (3) der Aufnahme nach Außen gewährleistet wird. Nach dem Verkleben ist die ursprüngliche Form ohne weitere Maßnahmen wieder gegeben, weil der Formkörper ja nicht völlig aufgeschnitten wurde.

Zum Verkleben können die Flächen durch Kontakt mit einer erhitzten Metalloberfläche oder mit einem möglichst präzis steuerbaren Heißluftstrom aufgeheizt und klebrig gemacht werden; das eigentliche Verkleben geschieht durch Aufeinanderpressen der erhitzten Flächen. Zum Verkleben wird also kein Klebstoff benötigt, sondern es werden die thermoplastischen Eigenschaften des verwendeten polymeren Grundkörpers zum Verkleben benutzt.

Es ist bevorzugt, die Ecken und Außenkanten des gebildeten Gel-Formkörpers (1) unter Verwendung von Schneidwerkzeugen zumindest teilweise zu fasen oder zu runden, um schalltechnisch günstige Bedingungen zu schaffen. Gegebenenfalls wird auch zumindest eine der schmalen Seitenflächen gerundet (4), wiederum unter Verwendung von Schneidwerkzeugen. Es können auch die Kanten von Boden- und Deckfläche paarweise nach außen gewölbt (5) werden, so dass die zugehörige schmale Seitenfläche gebogen erscheint. Auch diese Formgebung wird unter Verwendung von Schneidwerkzeugen erreicht.

Ein weiteres nicht beanspruchtes Verfahren zur Herstellung des erfindungsgemäßen Gel-Formkörpers (1) geht von einem formstabilen elastischen Gel Ein polymerer Grundkörper und eine geeignete niedermolekulare Flüssigkeit werden durch Spritzgießen in eine Form mit der gewünschten Gestalt als Gel-Formkörper (1) hergestellt, der nach Erkalten aus der Form herausgenommen wird. Die Aufnahme (2) entsteht in diesem Fall durch passend geformte Elemente der Spritzgussform.

Bei dem Gel-Formkörper (1) kann die Aufnahme (2) im Inneren mit einem nicht-formstabilen Ultraschallgel gefüllt werden. Das erleichtert das Einbringen von Ultraschallsender und -empfänger in den Gel-Formkörper (1).

Zudem kann der Gel-Formkörper (1) in eine flexible, biokompatible Schutzhülle (6) eingeführt werden, wodurch eine bequeme Handhabung des Gel-Formkörpers möglich wird.

Die Schutzhülle (6) kann mit einem nicht-formstabilen Ultraschallgel so gefüllt werden, dass der Gel-Formkörper (1) im Inneren der Hülle (6) ganz oder teilweise von dem Ultraschallgel umgeben ist. Die Schutzhülle (6) sollte so lang sein, dass der Gel-Formkörper (1) und das Ultraschallgel sicher in der Schutzhülle (6) Platz finden und nicht unabsichtlich herausrutschen bzw. ausfließen können. Bei der Verwendung der Vorrichtung, die in DE 10 2017 011 311.4 beschrieben ist, entspricht die Länge der Schutzhülle (6) zumindest der Länge der beiden starren Arme, die Teile des dort beschriebenen Handgriffs sind.

Führt man einen Ultraschallsender und einen Ultraschallempfänger (7) jeweils in einen entsprechenden Gel-Formkörper (1) ein, der sich in einer mit Ultraschallgel befüllten Schutzhülle (6) befindet, so erhält man eine Ultraschallmesseinheit, die unmittelbar für TAU-Messungen verwendet werden kann. Lediglich Luftblasen im Ultraschallgel müssen zuvor noch durch sanftes Ausstreichen mit dem Finger aus dem Messbereich entfernt werden.

Genannt wird schließlich die Verwendung eines Gel-Formkörpers für Ultraschallmessungen im ärztlichen (z.B. an Gelenken wie dem Knie) oder zahnärztlichen (insbesondere in der Mundhöhle) Bereich. Genannt wird weiterhin die Verwendung eines Gel-Formkörpers (1) als Einwegartikel im ärztlichen oder zahnärztlichen Bereich. Der Einsatz der Vorlaufstrecken gegenüber der Puls-Echo-Sonographie im Stand der Technik wird stark vereinfacht.

Bevorzugt ist die Verwendung des Gel-Formkörpers (1) bei Transmissions-Alveolar-Ultraschallmessungen zur Lokalisierung von Kavitäten im Kieferknochen, besonders bevorzugt bei einer Vorrichtung und einem Verfahren nach DE 10 2017 011 311.4.

Die Verwendung eines Gel-Formkörpers als Einwegartikel erfolgt aus hygienischen und aus messtechnischen Gründen. Wenn nach jedem Patienten die Gel-Formkörper (1) durch frische Gel-Formkörper ersetzt werden, ist optimale Hygiene gewährleistet. Außerdem wird dadurch so weit wie möglich vermieden, dass von außen nicht erkennbaren Schäden im Inneren der Gel-Formkörper die Messungen verfälschen können. Solche Schäden können durch zu viele aufeinander folgende Messungen entstehen. Durch Wechsel der Gel-Formkörper nach jedem Patienten wird aber die Wahrscheinlichkeit des Auftretens solcher Schäden vernachlässigbar klein gehalten.

## Patentansprüche

1. Verfahren zur Herstellung eines Gel-Formkörpers (1) zur Verwendung bei Ultraschallmessungen, wobei der Gel-Formkörper (1) elastisch ist, wobei im Inneren des Gel-Formkörpers eine Aufnahme (2) für einen für die Ultraschallmessungen vorgesehenen Sender und/oder Empfänger (7) vorgesehen ist, und wobei die Aufnahme (2) eine Öffnung (3) nach außen aufweist, durch die der Ultraschallsender und/oder -empfänger (7) in die Aufnahme (2) eingeführt werden kann, wobei der Gel-Formkörper für Ultraschallmessungen im ärztlichen oder zahnärztlichen Bereich und zur Einführung in die Mundhöhle eines Patienten, um dentale Kavitäten im Kieferknochen durch Transmissions-Alveolar- Ultraschallmessungen, TAU, nachzuweisen und zu lokalisieren, geeignet ist, **dadurch gekennzeichnet, dass** das Verfahren umfasst:
(i) Bereitstellen eines Ausgangsformkörpers, bestehend aus einem formstabilen, elastischen Gel, wobei der Ausgangsformkörper eine Bodenfläche, eine Deckfläche und gegenüberliegende Seitenflächen aufweist;
(ii) vollständiges oder teilweises Aufschneiden des Ausgangsformkörpers zwischen der Bodenfläche und der Deckfläche, und
(iii) Verkleben der zwei Schnittflächen miteinander an den gegenüberliegenden Seitenflächen so, dass der Gel-Formkörper (1) mit der Aufnahme (2) und der Öffnung (3) entsteht.

2. Verfahren zur Herstellung eines Gel-Formkörpers (1) nach Anspruch 1, wobei das Verkleben der zwei Schnittflächen umfasst:
Aufheizen der zwei Schnittflächen mit einer erhitzten Metallfläche oder mit einem steuerbaren Heißluftstrom; und anschließend
Pressen der zwei Schnittflächen zusammen.

3. Verfahren zur Herstellung eines Gel-Formkörpers (1) nach einem der Ansprüche 1 oder 2, **dadurch gekennzeichnet, dass**:
vorhandene Ecken und/oder Außenkanten zumindest teilweise gefast oder gerundet werden.

4. Verfahren zur Herstellung eines Gel-Formkörpers (1) nach einem der Ansprüche 1 oder 2, **dadurch gekennzeichnet, dass**:
eine der Öffnung gegenüberliegende Seitenfläche einen abgerundeten Abschnitt aufweist, wobei die Form des abgerundeten Abschnitts schon im Ausgangsformkörper vorgegeben ist oder nachträglich durch Beschneiden der Seitenfläche gebildet wird.

5. Verfahren zur Herstellung eines Gel-Formkörpers (1) nach einem der Ansprüche 1 bis 4, **dadurch gekennzeichnet, dass**:
die Aufnahme (2) im Inneren des Gel-Formkörpers (1) mit einem nicht-formstabilen Ultraschallgel gefüllt wird.

6. Verfahren zur Herstellung eines Gel-Formkörpers (1) nach einem der Ansprüche 1 bis 5, **dadurch gekennzeichnet, dass**:
der Gel-Formkörper (1) in eine flexible, vorzugsweise biokompatible Schutzhülle (6) eingeführt wird.

7. Verfahren zur Herstellung eines Gel-Formkörpers (1) nach Anspruch 6, **dadurch gekennzeichnet, dass** die Schutzhülle (6) mit einem nicht-formstabilen Ultraschallgel so gefüllt wird, dass der Gel-Formkörper (1) im Inneren der Hülle (6) ganz oder teilweise von dem nicht-formstabilen Ultraschallgel umgeben ist.

## Claims

1. Method for producing a gel moulded body (1) for use in ultrasound measurements, wherein the gel moulded body (1) is elastic, wherein a receptacle (2) for a transmitter and/or receiver (7) provided for the ultrasound measurements is provided in the interior of the gel moulded body, and wherein the receptacle (2) has an opening (3) to the outside, through which the ultrasound transmitter and/or receiver (7) can be introduced into the receptacle (2), wherein the gel moulded body is suitable for ultrasound measurements in the medical or dental field and for introduction into the oral cavity of a patient in order to detect and locate dental cavities in the jaw bone by transmission alveolar ultrasound measurements, TAU, **characterized in that** the method comprises:
(i) providing a starting moulded body consisting of a dimensionally stable, elastic gel, wherein the starting moulded body has a bottom surface, a top surface and opposite side surfaces; and
(ii) completely or partially cutting open the starting moulded body between the bottom surface and the top surface, and
(iii) adhesively bonding the two cut surfaces to one another at the opposite side surfaces such that the gel moulded body (1) with the receptacle (2) and the opening (3) is produced.

2. Method for producing a gel moulded body (1) according to Claim 1, wherein the adhesive bonding of the two cut surfaces comprises:
heating the two cut surfaces with a heated metal surface or with a controllable hot-air flow; and then
pressing the two cut surfaces together.

3. Method for producing a gel moulded body (1) according to either of Claims 1 and 2, **characterized in that**:
corners and/or outer edges which are present are at least partially chamfered or rounded.

4. Method for producing a gel moulded body (1) according to either of Claims 1 and 2, **characterized in that**:
a side surface opposite the opening has a rounded portion, wherein the shape of the rounded portion is already predefined in the starting moulded body or is formed subsequently by trimming the side surface.

5. Method for producing a gel moulded body (1) according to one of Claims 1 to 4,
**characterized in that**:
the receptacle (2) in the interior of the gel moulded body (1) is filled with a non-dimensionally stable ultrasound gel.

6. Method for producing a gel moulded body (1) according to one of Claims 1 to 5,
**characterized in that**:
the gel moulded body (1) is introduced into a flexible, preferably biocompatible protective sheath (6).

7. Method for producing a gel moulded body (1) according to Claim 6,
**characterized in that** the protective sheath (6) is filled with a non-dimensionally stable ultrasound gel in such a way that the gel moulded body (1) in the interior of the sheath (6) is completely or partially surrounded by the non-dimensionally stable ultrasound gel.

## Revendications

1. Procédé de fabrication d'un corps moulé en gel (1) à utiliser lors de mesures par ultrasons, dans lequel le corps moulé en gel (1) est élastique, dans lequel un logement (2) pour un émetteur et/ou récepteur (7) prévu pour les mesures par ultrasons est prévu à l'intérieur du corps moulé en gel, et dans lequel le logement (2) présente une ouverture (3) vers l'extérieur par laquelle l'émetteur et/ou récepteur à ultrasons (7) peut être introduit dans le logement (2), dans lequel le corps moulé en gel est adapté pour des mesures par ultrasons dans le domaine médical ou dentaire et pour l'introduction dans la cavité buccale d'un patient pour identifier et localiser des cavités dentaires dans l'os de la mâchoire par des mesures par ultrasonographie alvéolaire par transmission, TAU, **caractérisé en ce que** le procédé comprend :
(i) l'obtention d'un corps moulé de départ constitué d'un gel élastique indéformable, dans lequel le corps moulé de départ présente une surface de fond, une surface supérieure et des surfaces latérales opposées ;
(ii) la découpe totale ou partielle du corps moulé de départ entre la surface de fond et la surface supérieure, et
(iii) le collage des deux faces de coupe l'une à l'autre sur les faces latérales opposées, de manière à obtenir le corps moulé en gel (1) avec le logement (2) et l'ouverture (3).

2. Procédé de fabrication d'un corps moulé en gel (1) selon la revendication 1, dans lequel le collage des deux faces de coupe comprend :
le chauffage des deux faces de coupe avec une surface métallique chauffée ou avec un flux d'air chaud pouvant être commandé ; puis
la compression des deux faces de coupe.

3. Procédé de fabrication d'un corps moulé en gel (1) selon l'une des revendications 1 ou 2, **caractérisé en ce que** :
des coins et/ou des arêtes extérieures existants sont chanfreinés ou arrondis au moins en partie.

4. Procédé de fabrication d'un corps moulé en gel (1) selon l'une des revendications 1 ou 2, **caractérisé en ce que** :
une face latérale opposée à l'ouverture présente une section arrondie, dans lequel la forme de la section arrondie est déjà prédéfinie dans le corps moulé de départ ou est formée ultérieurement par découpe de la face latérale.

5. Procédé de fabrication d'un corps moulé en gel (1) selon l'une des revendications 1 à 4, **caractérisé en ce que** :
le logement (2) à l'intérieur du corps moulé en gel (1) est rempli d'un gel à ultrasons non indéformable.

6. Procédé de fabrication d'un corps moulé en gel (1) selon l'une des revendications 1 à 5, **caractérisé en ce que** :
le corps moulé en gel (1) est introduit dans une enveloppe de protection (6) flexible, de préférence biocompatible.

7. Procédé de fabrication d'un corps moulé en gel (1) selon la revendication 6, **caractérisé en ce que** l'enveloppe de protection (6) est remplie d'un gel à ultrasons non indéformable d'une manière telle que le corps moulé en gel (1) à l'intérieur de l'enveloppe (6) soit totalement ou partiellement entouré du gel à ultrasons non indéformable.
